# FASCICULE DE BREVET EUROPEEN

(11) **EP 0 527 853 B1**
(45) Date de publication et mention de la délivrance du brevet: **28.09.1994**
(21) Numéro de dépôt: 91909078.7
(22) Date de dépôt: 25.04.1991
(51) Int. Cl.: A61F 13/15

(54) **PROCEDE DE FABRICATION EN CONTINU D'ARTICLES D'HYGIENE TELS QUE DES COUCHES-CULOTTES, ET ARTICLE D'HYGIENE AINSI FABRIQUE**
VERFAHREN ZUR KONTINUIERLICHEN HERSTELLUNG VON HYGIENEARTIKELN INSBESONDERE WINDELN, UND SO HERGESTELLTE HYGIENEARTIKEL
METHOD FOR CONTINUOUSLY PRODUCING SANITARY ITEMS SUCH AS NAPPIES, AND SANITARY ARTICLE THEREBY PRODUCED

(30) Priorité: 07.05.1990 FR 9005732
(43) Date de publication de la demande: 24.02.1993
(73) Titulaire: PEAUDOUCE, 59126 Linselles (FR)
(72) Inventeur: LECLERCQ, Maurice, Pierre 1132, route Nationale, F-80132 Abbeville (FR); DUSSAUD, Jacques, F-59110 La Madeleine (FR)
(74) Mandataire: Casalonga, Axel
(86) Numéro de dépôt international: FR9100341
(87) Numéro de publication internationale: WO9116870

(56) Documents cités:
- EP-A- 0 170 922
- FR-A- 2 588 285
- US-A- 3 746 607
- US-A- 4 273 815
- US-A- 4 351 784
- US-A- 4 771 483

## Description

La présente invention se rapporte à un procédé de fabrication en continu d'articles d'hygiène tels que des couches-culottes, du type comprenant un coussin absorbant disposé entre une feuille extérieure imperméable aux liquides, et une feuille intérieure perméable aux liquides. L'invention se rapporte également à un article d'hygiène ainsi fabriqué

Suivant les procédés usuels de fabrication en continu de tels articles, un film continu de matière plastique, par exemple de polyéthylène, est déroulé d'une bobine et enduit de lignes ou bandes de colle du type thermofusible ("hot melt"), des coussins absorbants individuels espacés sont déposés à intervalles sur le film puis une bande continue de matière perméable, par exemple une bande de non-tissé, est appliquée sur le film et sur les coussins de manière à adhérer à ladite feuille au moins le long des bords longitudinaux de cette dernière. Lorsque ces articles sont munies d'éléments élastiques longitudinaux, des éléments élastiques continus sont enduits de colle au moins par intervalles et sont déposés à l'état tendu sur le film avant l'application sur ce dernier de la bande perméable.

Ces procédés connus présentent un certain nombre d'inconvénients.

Ainsi, l'utilisation de bobines de film de polyéthylène impose des contraintes de manipulation et implique des pertes de temps lors des changements de bobines. La liaison entre eux des différents éléments constitutifs des articles d'hygiène (feuille extérieure, feuille intérieure, éléments élastiques et autres) entraîne une consommation de colle relativement importante et impose des contraintes non négligeables (limitation de vitesse, précision d'application, etc.).

Par ailleurs, sur les couches-culottes fabriquées suivant les procédés usuels, sur lesquelles la feuille extérieure n'adhère aux coussins absorbants que suivant des lignes ou bandes espacées, la résistance et notamment la résistance à la déchirure de la feuille extérieure de la couche-culotte est souvent insuffisante, pour permettre, après un premier collage, un décollage des attaches adhésives en vue de leur repositionnement.

La présente invention a pour objet un procédé de fabrication en continu d'articles d'hygiène tels que des couches-culottes supprimant les contraintes dues à l'utilisation de film de polyéthylène en bobines pour constituer la feuille extérieure imperméable aux liquides des articles d'hygiène et supprimant ou pour le moins réduisant considérablement l'utilisation de colle par exemple du type thermofusible pour lier entre eux les éléments constitutifs des articles d'hygiène, ainsi que les contraintes résultant de l'utilisation de colle. L'invention a également pour objet un procédé de fabrication en continu d'articles d'hygiène permettant d'obtenir des articles d'hygiène de résistance améliorée.

L'invention a par ailleurs pour objet des articles d'hygiène tels que des couches-culottes fabriqués à un coût réduit.

Suivant le procédé conforme à l'invention de fabrication en continu d'articles d'hygiène tels que des couches-culottes, comprenant un coussin absorbant disposé entre une feuille extérieure imperméable aux liquides et une feuille intérieure perméable aux liquides, on dépose des coussins absorbants individuels par intervalles sur une bande continue perméable aux liquides, déroulée d'une bobine. On extrude ensuite et dépose un film de matière plastique à l'état non encore solidifié sur ladite bande et sur lesdits coussins, en appliquant ledit film contre la bande et contre le coussin pour l'y faire adhérer. Après solidification dudit film, on découpe la structure composite transversalement en articles d'hygiène individuels dans la zone située entre les coussins successifs.

Le film extrudé étant déposé et appliqué sur la bande et sur les coussins avant sa solidification, il y adhère sans qu'il soit nécessaire d'utiliser de la colle comme selon les procédés connus. De plus, le film extrudé se trouve lié de façon continue, par liaison directe aux coussins absorbants et à la bande sur toute la surface de contact et non pas seulement suivant des lignes de collage espacées comme suivant les procédés connus. De ce fait, la cohésion et en particulier la résistance à la déchirure de la feuille extérieure des articles d'hygiène se trouve renforcée par le coussin absorbant. Ainsi, il est possible de coller et de décoller les attaches adhésives sur cette zone de la feuille extérieure, en vue du repositionnement des attaches, même en cas d'utilisation pour la feuille extérieure d'un film relativement mince.

Pour la fabrication en continu d'articles d'hygiène comportant des éléments élastiques longitudinaux fixés à l'état tendu à la feuille extérieure, de part et d'autre du coussin absorbant, il est proposé, suivant le procédé conforme à l'invention, qu'on dépose des éléments élastiques à l'état tendu sur ladite bande avant d'extruder et de déposer le film de matière plastique sur la bande et sur les coussins, de manière que ledit film adhère également auxdits éléments élastiques lors de sa solidification.

Par conséquent, la fixation des éléments élastiques s'effectue également sans utilisation de colle.

Suivant les procédés de fabrication connus de couches-culottes comportant des éléments élastiques longitudinaux qui ne sont fixés à la feuille extérieure imperméable que sur une partie de leur longueur, à savoir dans la zone d'entre-jambes des couches-culottes, il est connu d'encoller des éléments élastiques continus par intervalles de sorte que lors de la coupe transversale de la structure composite comprenant une bande imperméable, une bande perméable et des coussins absorbants espacés disposés entre lesdites deux bandes, les éléments élastiques se contractent librement dans leurs parties non encollées. Suivant le procédé conforme à l'invention, il est prévu, en vue de la fabrication d'articles d'hygiène comportant des éléments élastiques fixés à la feuille extérieure uniquement sur une partie de leur longueur, de munir les éléments élastiques continus par intervalle d'une enduction de matière anti-adhésive pour les empêcher d'adhérer dans ces zones au film extrudé et déposé à l'état non encore solidifié sur la bande, les coussins absorbants et lesdits éléments élastiques continus. Ainsi, lors de la coupe transversale de la structure composite en articles individuels, ces éléments élastiques peuvent se contracter librement dans leurs parties munies d'une enduction de matière anti-adhésive, le film n'adhérant pas aux éléments élastiques dans ces parties.

Suivant une autre caractéristique du procédé conforme à l'invention, concernant la fixation des attaches adhésives pour la fermeture des couches-culottes, on dépose et fixe les attaches adhésives par collage par leurs parties de fixation sur la bande perméable avant d'extruder et de déposer le film à l'état non encore solidifié sur ladite bande et les parties de fixation des attaches adhésives. Ainsi, sur les couches-culottes terminées, les attaches adhésives se trouvent solidement fixées entre, et adhèrent à la feuille intérieure perméable, par exemple en non-tissé, et la feuille extérieure imperméable, par exemple en polyéthylène.

Par ailleurs, pour fabriquer des couches-culottes comprenant des éléments transversaux de ceinture, éventuellement élastifiés, ces éléments transversaux sont généralement fixés par collage à la feuille extérieure imperméable. Suivant le procédé conforme à l'invention, il est par contre possible de fixer ces éléments transversaux de ceinture sans utiliser de colle, en déposant lesdits éléments de ceinture sur la bande avant d'extruder et de déposer sur la bande ledit film à l'état non encore solidifié. De ce fait, les éléments transversaux de ceinture se trouvent solidement fixés sans colle à ce film, donc à la feuille extérieure imperméable sur la couche-culotte.

En se référant aux dessins schématiques annexés, on va décrire ci-après plus en détail l'invention; sur les dessins :
la figure 1 représente un schéma de principe du procédé conforme à l'invention de fabrication de couches-culottes;
la figure 2 est une coupe transversale suivant II-II de la figure 1, à plus grande échelle, d'une couche-culotte réalisée suivant l'invention;
la figure 3 représente un schéma de principe d'un autre mode de réalisation du procédé conforme à l'invention.

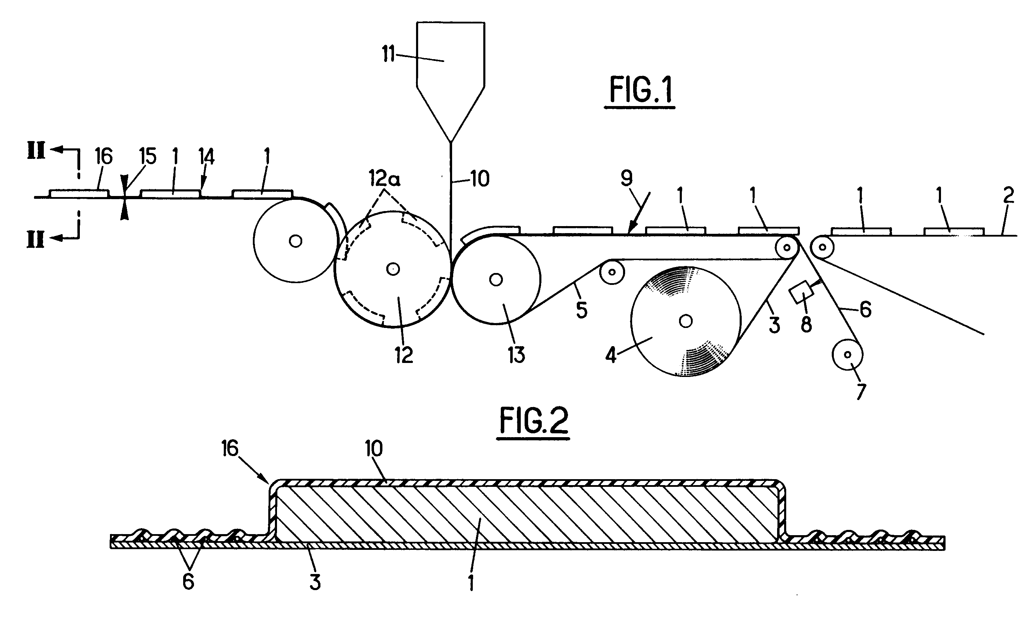

Le procédé conforme à l'invention tel qu'illustré par la figure 1 consiste à amener des coussins absorbants 1, par exemple à base de fluff de cellulose, véhiculés par un transporteur 2, sur une bande 3 perméable, par exemple une bande de non-tissé, déroulée d'une bobine 4, la bande 3 étant soutenu par un transporteur 5, par exemple une bande transporteuse aspirante. En même temps que les coussins absorbants 1, des éléments élastiques 6 continus retirés d'une bobine 7 sont, après avoir été munis par intermittence d'une enduction de matière anti-adhésive, par exemple de silicone, à un poste d'enduction 8, déposés à l'état tendu sur la bande 3, dans la direction longitudinale de cette dernière, de part et d'autre des coussins absorbants 1.

Au poste 9, des attaches adhésives peuvent être déposées sur la bande 5 de manière que leurs parties de fixation recouvrent les bords longitudinaux de la bande 3, au voisinage d'une extrémité de chaque coussin 1.

A l'extrémité du trajet de transport défini par la bande transporteuse 5, un film 10 de matière thermoplastique, par exemple de polyéthylène, extrudé par une filière 11, est déposé sur la bande 3 ainsi que sur tous les autres éléments (coussins absorbants 1, éléments élastiques 6, parties de fixation des attaches adhésives) préalablement déposés sur la bande 3. Le film 10 qui peut avoir par exemple une épaisseur comprise entre 5 et 30µm est appliqué, à l'état non encore solidifié, contre la bande 3 et les éléments portés par cette bande, entre un cylindre refroidisseur 12 muni d'alvéoles 12a épousant la forme des coussins 1 et un cylindre de contre-pression 13 également refroidi servant en même temps de cylindre de renvoi pour la bande transporteuse 5.

De préférence, la largeur de la filière 11 est réglable, ce qui permet d'extruder le film 10 avec une largeur correspondant à la largeur des articles d'hygiène à fabriquer. Après refroidissement du film 10, ce dernier adhère sur une face des coussins absorbants 1 ainsi que tout le pourtour des bords de ces derniers, quelle que soit la forme des coussins (rectangulaire, en forme de sablier), formant ainsi une barrière d'étanchéité aux liquides. De plus, le film 10 adhère à la bande 3 autour des coussins 1, sur toute la surface de la bande 3 non recouverte par les coussins 1.

Le film 10 adhère de plus aux parties des éléments élastiques 6 non munies d'une enduction de matière anti-adhésive. Enfin, le film 10 adhère aux parties de fixation des attaches adhésives déposées en 9 sur la bande 3.

Après solidification du film 10 sur le cylindre 12, la structure composite 14 formée de la bande 3, des coussins absorbants 1, des éléments élastiques 6 et des attaches adhésives est amenée à un poste de découpage transversal 15 auquel la structure 14 est découpée en articles d'hygiène 16 individuels dans la zone comprise entre chaque fois deux coussins absorbants 1 successifs.

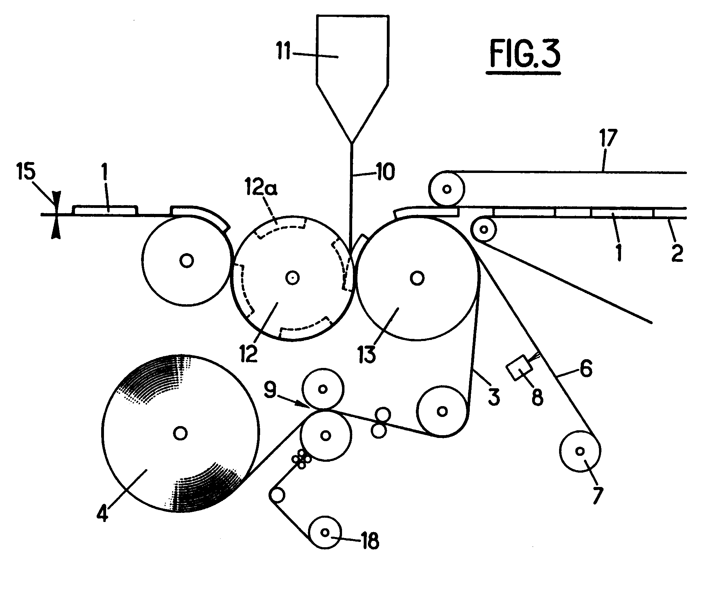

Le procédé tel qu'illustré par la figure 3 diffère du procédé selon la figure 1 par le fait que les coussins 1 amenés par le transporteur 2 en étant maintenu par une bande supérieure 17, arrivent non pas sur une bande transporteuse 5, mais directement sur le cylindre de contre-pression 13 sur lequel arrive également de la bobine 4 la bande 3 sur laquelle ont été préalablement collées en 9 les attaches adhésives provenant d'une bobine 18. Le cylindre de contre-pression coopérant avec le cylindre de refroidissement 12 présentant des alvéoles 12a, comporte un ou plusieurs segments aspirants non représentés qui maintiennent les coussins par rapport à la bande 3 jusqu'à leur contact avec le film 10 extrudé par la filière 11.

Ainsi, la pose des attaches adhésives, au lieu de se faire au poste 9 préalablement à la dépose du film 10, pourrait également se faire après dépose du film 10, les attaches adhésives étant alors fixées de la manière habituelle par collage.

Par ailleurs, il serait également possible de déposer sur la bande perméable 3, préalablement au dépôt du film 10, des éléments transversaux de ceinture, éventuellement élastiques et thermorétractables, de manière que ces derniers adhèrent également à la feuille extérieure (10) des couches-culottes sans utilisation de colle. En cas d'utilisation d'éléments transversaux de ceinture élastiques posés à l'état tendu, il serait nécessaire de prévoir un collage provisoire de ces éléments, au moins à leurs extrémités.

Le film 10, au lieu d'être constitué par du polyéthylène pouvant être extrudé à une température de l'ordre de 300°C, peut être constitué par toute autre matière thermoplastique appropriée, par exemple EVA (copolymère éthylène/acétate de vinyle) ou EBA (copolymère éthylène/acrylate de butyle), ce film pouvant être de préférence perméable à la vapeur d'eau. Ce film qui peut être coloré dans la masse peut avoir une épaisseur variable (dans le sens transversal et/ou dans le sens longitudinal) et présenter des zones élastifiées, par exemple pour former la zone de ceinture des couches-culottes).

De même, la bande 3 perméable aux liquides pourrait être constituée par une matière perméable autre que du non-tissé, par exemple une feuille perforée.

Les coussins absorbants 1 qui peuvent également être déposés sur la bande 3 selon plusieurs lignes parallèles, ce qui permet d'augmenter en conséquence la capacité de production peuvent être constitués par exemple par des fibres de cellulose de pâte fluff ou tout autre matériau, par exemple des fibres thermofusibles qui, par fusion partielle, permettent d'améliorer la cohésion interne du coussin, ou des fibres minérales, ou encore un mélange de fibres thermofusibles et ou de fibres minérales avec des fibres de cellulose. Par ailleurs, d'autres constituants tels que des matériaux superabsorbants sous forme de particules ou de fibres peuvent également être incorporés aux coussins absorbants.

Les éléments élastiques 6 peuvent être des éléments multibrins, par exemple à quatre brins comme représenté sur la figure 2, ou également des éléments élastiques individuels, à deux brins, etc. Pour empêcher la feuille 10 d'adhérer aux éléments élastiques dans les parties des éléments élastiques destinées à se contracter après sectionnement de la structure composite 14 au poste de coupe 15, ces éléments élastiques peuvent être munis d'une enduction de silicone ou de toute autre matière anti-adhésive appropriée.

Enfin, l'invention est applicable à la fabrication d'articles d'hygiène de tous types (couches-culottes pour enfants et adultes, garnitures pour incontinents, serviettes hygiéniques) comprenant un coussin absorbant disposé entre une feuille imperméable aux liquides et une feuille perméable aux liquides, avec ou sans éléments élastiques.

## Revendications

1. Procédé de fabrication en continu d'articles d'hygiène tels que des couches-culottes, comprenant un coussin absorbant disposé entre une feuille extérieure imperméable aux liquides et une feuille intérieure perméable aux liquides, caractérisé par le fait qu'on dépose des coussins absorbants individuels (1) à intervalles sur une bande continue (3) perméable aux liquides, déroulée d'une bobine (4), et qu'on extrude et dépose un film de matière plastique (10) à l'état non encore solidifié sur ladite bande et sur lesdits coussins, en appliquant ledit film contre la bande et contre les coussins pour l'y faire adhérer, et qu'après solidification dudit film, on coupe transversalement la bande composite ainsi formée, dans la zone située entre les coussins absorbants.

2. Procédé suivant la revendication 1, pour la fabrication d'articles d'hygiène comportant des éléments élastiques longitudinaux fixés à l'état tendu à la feuille extérieure, de part et d'autre des coussins absorbants, caractérisé par le fait qu'on dépose des éléments élastiques continus (6) à l'état tendu sur ladite bande (3) avant d'extruder et de déposer ledit film (10) sur la bande de manière que ledit film adhère également auxdits éléments élastiques lors de sa solidification.

3. Procédé suivant la revendication 2, pour la fabrication d'articles d'hygiène dans lesquels lesdits éléments élastiques (6) ne sont fixés à ladite feuille que sur une partie de leur longueur, caractérisé par le fait qu'on munit lesdits éléments élastiques continus (6) par intervalles d'une enduction de matière anti-adhésive (8) pour les empêcher d'adhérer dans ces zones au film extrudé et déposé à l'état non encore solidifié sur ladite bande, lesdits coussins et lesdits éléments élastiques.

4. Procédé suivant l'une quelconque des revendications précédentes, pour la fabrication de couches-culottes comprenant, en outre, des attaches adhésives pour la fermeture de la couche-culotte, caractérisé par le fait qu'on dépose et fixe les attaches adhésives par collage sur la bande (3) avant d'extruder et de déposer ledit film (10) sur la bande de manière que ledit film adhère également auxdites attaches lors de sa solidification.

5. Procédé suivant l'une quelconque des revendications précédentes, pour la fabrication de couches-culottes comprenant, en outre, des éléments transversaux de ceinture, éventuellement élastifiés, caractérisé par le fait qu'on dépose lesdits éléments de ceinture sur la bande (3) avant d'extruder et de déposer ledit film (10) sur la bande de manière que ledit film adhère également auxdits éléments de ceinture lors de sa solidification.

6. Article d'hygiène tel que couche-culotte, comprenant un coussin absorbant (1) disposé entre une feuille extérieure (10) imperméable aux liquides et une feuille intérieure (3) perméable aux liquides, caractérisé par le fait que la feuille extérieure (10) adhère au coussin absorbant (1) et à la feuille intérieure (3) autour du coussin de façon continue par contact direct, sur toute la surface de contact, sans adjonction de colle.

7. Article d'hygiène suivant la revendication 6, comprenant en outre des éléments élastiques longitudinaux (6) fixés à l'état tendu à la feuille extérieure (10), de part et d'autre du coussin absorbant (1), caractérisé par le fait que les éléments élastiques (6) sont fixés sans adjonction de colle à la feuille extérieure.

8. Article d'hygiène suivant la revendication 6 ou 7, comprenant, en outre, au moins un élément transversal de ceinture, éventuellement élastifié, caractérisé par le fait que ledit élément transversal de ceinture est fixé sans adjonction de colle à la feuille extérieure (10).

9. Article d'hygiène suivant l'une quelconque des revendications 6 à 8, caractérisé par le fait que la feuille extérieure (10) est perméable à la vapeur.

## Claims

1. Process for the continuous manufacture of articles of hygiene such as nappy-pants, comprising an absorbent pad arranged between a liquid-impervious outer sheet and a liquid-permeable inner sheet, characterised in that individual absorbent pads (1) are deposited at intervals onto a liquid-permeable continuous strip (3) unwound from a reel (4) and in that a film of plastic material (10) is extruded and deposited in the state where it has not yet solidified onto the said strip and onto the said pads, by applying the said film against the strip and against the pads to make it adhere thereto and in that, after solidification of the said film, the composite stripo thus formed is cut transversely in the region situated between the absorbent pads.

2. Process according to Claim 1, for the manufacture of articles of hygiene comprising lengthwise elastic members attached in the stretched state to the outer sheet on both sides of the absorbent pads, characterised in that continuous elastic members (6) are deposited in the stretched state onto the said strip (3) before the said film (10) is extruded and deposited onto the strip so that the said film also adheres to the said elastic members when it solidifies.

3. Process according to Claim 2, for the manufacture of articles of hygiene in which the said elastic members (6) are attached to the said sheet only over a part of their length, characterised in that the said continuous elastic members (6) are provided at intervals with a coating of antiadhesive material (8) to prevent them from adhering in these regions to the film which is extruded and deposited in the state where it has not yet solidified onto the said strip, the said pads and the said elastic members.

4. Process according to any one of the preceding claims, for the manufacture of nappy-pants additionally comprising adhesive fastenings for closing the nappy-pants characterised in that adhesive fastenings are deposited and attached by adhesive bonding onto the strip (3) before the said film (10) is extruded and deposited onto the strip so that the said film also adheres to the said fastenings when it solidifies.

5. Process according to any one of the preceding claims, for the manufacture of nappy-pants additionally comprising optionally elasticated transverse belt members, characterised in that the said belt members are deposited onto the strip (3) before the said film (10) is extruded and deposited onto the strip so that the said film also adheres to the said belt members when it solidifies.

6. Article of hygiene such as nappy-pants, comprising an absorbent pad (1) arranged between a liquid-impervious outer sheet (10) and a liquid-permeable inner sheet (3), characterised in that the outer sheet (10) adheres to the absorbent pad (1) and to the inner sheet (3) around the pad continuously by direct contact over the whole area of contact, without addition of adhesive.

7. Article of hygiene according to Claim 6, additionally comprising lengthwise elastic members (6) attached in the stretched state to the outer sheet (10) on both sides of the absorbent pad (1), characterised in that the elastic members (6) are fastened without addition of adhesive to the outer sheet.

8. Article of hygiene according to Claim 6 or 7, additionally comprising at least one optionally elasticated transverse belt member, characterised in that the said transverse belt member is attached without addition of adhesive to the outer sheet (10).

9. Article of hygiene according to any one of Claims 6 to 8, characterised in that the outer sheet (10) is permeable to vapour.

## Patentansprüche

1. Verfahren zur kontinuierlichen Herstellung von Hygieneartikeln, wie etwa Höschenwindeln, die ein absorbierendes Kissen aufweisen, das zwischen einer äußeren, für Flüssigkeiten undurchlässigen Folie und einer inneren, für Flüssigkeiten durchlässigen Folie angebracht ist, dadurch gekennzeichnet, daß man einzelne absorbierende Kissen (1) in Zwischenabständen auf ein für Flüssigkeiten durchlässigen kontinuierlichen Band (3) legt, daß von einer Spule (4) abgewickelt wird, und daß man einen Kunststoffilm (10) extrudiert in einem noch nicht verfestigten Zustand auf das Band und auf die Kissen legt, indem man den Film gegen das Band und gegen die Kissen drückt, um es daran anzuheften, und daß man nach der Verfestigung den Films das so gebildete, zusammengesetzte Band quer in der Zone zwischen den absorbierenden Kissen durchschneidet.

2. Verfahren nach Anspruch 1, zur Herstellung von Hygieneartikeln, die zu beiden Seiten der absorbierenden Kissen längsgerichtete elastische Elemente aufweisen, welche im genannten Zustand auf der äußeren Folie befestigt sind, dadurch gekennzeichnet, daß man vor dem Extrudieren und Auflegen des Filmes (10) auf das Band elastische kontinuierliche Elemente (6) im gespannten Zustand auf das Band (3) legt, derart, daß sich der Film bei seiner Verfestigung auch an den elastischen Elementen anheftet.

3. Verfahren nach Anspruch 2 zur Herstellung von Hygieneartikeln, bei denen die elastischen Elemente (6) an der Folie nur über einen Teil ihrer Länge befestigt sind, dadurch gekennzeichnet, daß man die kontinuierlichen elastischen Elemente (6) in Abständen mit einer Beschichtung aus einem antiadhäsiven Material versieht, um sie daran zu hindern, in diesen Zonen die Kissen und elastischen Elemente am extrudierten und im noch nicht verfestigten Zustand auf das Band gelegten Film anzuheften.

4. Verfahren nach einem beliebigen vorhergehenden Ansprüche zur Herstellung von Höschenwindeln, die weiter Klebeverbinder zum Schließen der Höschenwindel aufweisen, dadurch gekennzeichnet, daß man vor dem Extrudieren und Auflegen des Films (10) auf das Band die Klebeverbinder auf dem Band (3) durch Klebung befestigt, derart, daß sich der Film bei seiner Verfestigung auch an den Klebeverbindern anheftet.

5. Verfahren nach einem beliebigen vorhergehenden Anspruch zur Herstellung von Höschenwindeln, die weiter querverlaufende Gürtelelemente, welche eventuell elastischgemacht sind, aufweisen, dadurch gekennzeichnet, daß man vor dem Extrudieren und Auflegen des Films (10) auf das Band die Gürtelelemente auf das Band (3) legt, derart, daß sich der Film bei seiner verfestigung auch an die Gürtelelemente anheftet.

6. Hygieneartikel, wie etwa eine Höschenwindel, der ein absorbierendes Kissen aufweist, welches zwischen einer äußeren Folie (10), die für Flüssigkeiten undurchlässig ist, und einer inneren Folie (3), die für Flüssigkeiten durchlässig ist, angeordnet ist, dadurch gekennzeichnet, daß die äußere Folie (10) am absorbierenden Kissen (1) und an der inneren Folie (3) um das Kissen herum durch engen Kontakt kontinuierlich haftet, und zwar über die gesamte Kontaktfläche, ohne Zugabe von Klebstoff.

7. Hygieneartikel nach Anspruch 6, der weiter längagerichtete, elastische Elemente (6) aufweist, die im gespannten Zustand zu beiden Seiten des absorbierenden Kissens (1) befestigt sind, dadurch gekennzeichnet, daß die elastischen Elements ohne Zugabe von Klebstoff an der äußeren Folie befestigt sind.

8. Hygieneartikel nach Anspruch 6 oder 7, der weiter mindestens ein querverlaufendes Gürtelelement, das eventuell elastischgemacht ist, aufweist, dadurch gekennzeichnet, daß das querverlaufende Gürtelelement ohne Zugabe von Klebstoff an der äußeren Folie (10) befestigt ist.

9. Hygieneartikel nach einem beliebigen Anspruch 6 bis 8, dadurch gekennzeichnet, daß die äußere Folie (10) für Dampf durchlässig ist.
